(19) Europäisches Patentamt
European Patent Office
Office européen des brevets

(11) Veröffentlichungsnummer: **0 157 088**
**B1**

(12) # EUROPÄISCHE PATENTSCHRIFT

(45) Veröffentlichungstag der Patentschrift: **14.01.87**

(51) Int. Cl.⁴: **C 07 C 127/24**

(21) Anmeldenummer: **85100574.4**

(22) Anmeldetag: **21.01.85**

(54) **Verfahren zur Herstellung von Polyisocyanaten mit Biuretstruktur.**

(30) Priorität: **31.01.84 DE 3403278**

(43) Veröffentlichungstag der Anmeldung:
**09.10.85 Patentblatt 85/41**

(45) Bekanntmachung des Hinweises auf die Patenterteilung:
**14.01.87 Patentblatt 87/3**

(84) Benannte Vertragsstaaten:
**BE DE FR GB IT NL**

(56) Entgegenhaltungen:
**Keine Entgegenhaltungen**

(73) Patentinhaber: **BAYER AG, Konzernverwaltung RP Patentabteilung, D-5090 Leverkusen 1 Bayerwerk (DE)**

(72) Erfinder: **König, Klaus, Dr., Heymannstrasse 50, D-5090 Leverkusen 1 (DE)**
Erfinder: **Pedain, Josef, Dr., Haferkamp 6, D-5000 Köln 80 (DE)**
Erfinder: **Woynar, Helmut, Dr., Ahornweg 7, D-4047 Dormagen (DE)**

**EP 0 157 088 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1987

**Beschreibung**

Die vorliegende Erfindung beschreibt ein verbessertes Verfahren zur Herstellung von Polyisocyanaten mit Biuretstruktur, die ausgezeichnete Farbqualität und gute Monomerenstabilität aufweisen, durch Umsetzung von aliphatischen Diisocyanaten mit Wasser in Gegenwart spezieller Carbonsäuren und/oder deren Anhydride als Katalysatoren.

Aliphatische Polyisocyanate mit Biuretstruktur, insbesondere auf der Basis von Hexamethylendiisocyanat, haben weltweit für die Herstellung lichtechter und extrem witterungsbeständiger Lacke mit höchster Glanzhaltung technische Bedeutung erlangt. Für den Einsatz auf diesem Gebiet, insbesondere für klare und weiß pigmentierte Beschichtungen, werden vom Markt wenig bis nicht gefärbte produkte verlangt. Weiterhin ist für eine gefahrlose Verarbeitung ein möglichst geringer Anteil an monomeren Diisocyanaten anzustreben, der sich auch bei längerer Lagerung nicht erhöht. Aufgrund toxikologischer Untersuchungen ist eine gefahrlose Verarbeitung bis zu einem maximalen Gehalt von 0,7 % an monomerem Diisocyanat gegeben, sofern die bei der Lackverarbeitung üblichen Schutzvorschriften eingehalten werden. Der vorgenannte Grenzwert hat in die Literatur (z.B. Merkblatt "PUR-Anstrichstoffe" des Hauptverbandes der deutschen gewerblichen Berufsgenossenschaft sowie "Polyurethane Report", der Paintmakers Association) Eingang gefunden.

Für die Herstellung solcher Polyisocyanate ist im Laufe der Zeit eine Vielzahl von Verfahren bekannt geworden, die jedoch alle mit speziellen Problemen und Nachteilen behaftet sind und die obengenannten Forderungen an das Produkt nicht oder nur unvollständig erfüllen. Beispielsweise sind folgende Verfahren beschrieben:

- Synthese aus Diisocyanaten und Wasser, gegebenenfalls in Gegenwart von Katalysatoren; siehe DE-PS 1 101 394, DE-OS 1 668 377, DE-OS 2 308 015, GB-PS 889 050, GB-PS 1 399 228, DDR-PS 140 744.
- Synthese aus Diisocyanaten und Wasser in Gegenwart eines Lösungsmittels bzw. eines Lösungsmittelgemisches, siehe DE-OS 2 808 801, DE-OS 3 030 655.
- Synthese aus Diisocyanaten und Wasser, wobei das Wasser dampfförmig zur Reaktion gebracht wird, siehe DE-OS 2 918 739.
- Synthese aus Diisocyanaten und Schwefelwasserstoff, gegebenenfalls in Anwesenheit von Katalysatoren, siehe DE-AS 1 165 850.
- Synthese aus Diisocyanaten und Ammoniak bzw. Ammoniak-Wasser-Gemischen, gegebenenfalls in Anwesenheit von Katalysatoren, siehe DE-AS 1 227 003.
- Synthese aus Diisocyanaten und Aminen, siehe DE-PS 1 165 580, DE-PS 1 174 759, DE-OS 1 568 017, DE-OS 1 963 190, DE-OS 2 010 887, DE-OS 2 261 065, DE-AS 2 438 258, US-P 3 824 266, DE-AS 2 609 995, DE-OS 2 803 103, DE-PS 883 504, GB-PS 1 263 609, siehe auch Angew. Chem. 72, S. 1002.
- Synthese aus Diisocyanaten und Amin/Alkohol-Gemischen, siehe DE-OS 2 654 745.
- Synthese aus Diisocyanaten und ω,ω'-Diaminopolyethern, siehe DE-OS 1 570 632, DE-AS 1 215 365.
- Synthese aus Diisocyanaten und substituierten Harnstoffen, siehe DE-PS 1 101 394, DE-AS 1 227 004.
- Synthese aus Diisocyanaten und tertiären Alkoholen, gegebenenfalls in Anwesenheit von Katalysatoren, siehe DE-AS 1 543 178, DE-AS 1 931 055, DE-OS 2 308 015.
- Synthese aus Diisocyanaten und Ameisensäure, siehe DE-PS 1 174 760, DE-OS 2 308 015, DE-OS 2 437 130.
- Synthese aus Diisocyanaten und Aldoximen, siehe DE-OS 3 007 679.

Die bekannten Verfahren, bei denen die Diisocyanate direkt mit Wasser zur Reaktion gebracht werden, sind infolge der Inhomogenität des Reaktionsgemisches schwer zu beherrschen. So kann es zur Ausbildung von äußerst schwerlöslichem Polyharnstoff kommen, zu dessen Auflösung hohe Temperaturen über einen langen Zeitraum notwendig sind und wodurch die Farbe des Produktes negativ beeinflußt wird. Selbst dann bleibt unter Umständen ein Teil dieser Polyharnstoffe als schwer filtrierbarer Niederschlag ungelöst und muß vor der Weiterverarbeitung aufwendig abgetrennt werden. Weiterhin können sich infolge der Wasserdampfflüchtigkeit der meisten Diisocyanate Harnstoffablagerungen im Dampfraum des Reaktionsgefäßes bilden. Dies gilt auch für Verfahren, bei denen Wasser dampfförmig eingesetzt wird.

Diese Ablagerungen können bei den bisher bekannten Verfahren, die Wasser als Biuretisierungsmittel verwenden, nur dann vermieden werden, wenn Lösungsmittel, bzw. Lösungsmittelgemische zur Homogenisierung des Reaktionsgemischen eingesetzt werden. Diese Verfahren sind jedoch mit verschiedenen Nachteilen verbunden. Zu einen sind große Lösungsmittelmengen notwendig, die in einem weiteren Verfahrensschritt zum fertigen Produkt durch Destillation entfernt werden müssen, zum anderen sind für farblose Produkte ganz spezielle Lösungsmittelgemische aus Glykoletheracetaten und Phosphorsäureestern notwendig. Weiterhin sind bei diesen Verfahren Reaktionstemperaturen von mindestens 140°C notwendig, um das intermediäre Ausfällen von unlöslichen Harnstoffen zu vermeiden. Bilden sich dennoch z.B. infolge niedrigerer Reaktionstemperaturen, solche Ausfällungen, sind, wie auch bei den Verfahren, bei denen Wasser ohne Lösungsmittel eingesetzt wird, Temperaturen von 160°C und mehr notwendig, um klare Produkte zu erhalten. Diese Temperaturbelastung führt zu einem vermehrten Auftreten von Nebenprodukten sowie zu einer deutlichen Verschlechterung der Farbqualität.

Ohne Lösungsmittel können Verfahren durchgeführt werden, bei denen Wasser während der Reaktion aus einer wasserabspaltenden Verbindung freigesetzt wird. Hierzu gehört insbesondere auch das technisch bedeutsame Verfahren mit tert.-Butanol und anderen tert. Alkoholen als Biuretisierungsmittel. Dieses Verfahren erfordert jedoch ebenfalls Temperaturen von ca. 180°C mit allen bereits angesprochenen Nachteilen

für die Qualität des Produktes. Weiterhin beinhaltet dieses Verfahren den Verlust des Biuretisierungsmittels unter Freisetzung brennbarer Gase (Isobuten).

Die Umsetzung von Diisocyanaten mit Aldoximen ist ebenfalls gekennzeichnet durch den Verlust des schwer zugänglichen Biuretisierungsmittels und das Auftreten von leichtflüchtigen Nebenprodukten (Nitrile), die nicht wiederverwendet werden können.

Die Reaktion von Diisocyanaten mit Schwefelwasserstoff liefert das giftige, leicht siedende Kohlenoxisulfid, das ebenfalls nicht wieder in den Prozeß zurückgeführt werden kann und aufwendig entsorgt werden muß.

Allen bisher genannten Verfahren ist gemeinsam, daß ein Teil des Diisocyanats durch Reaktion mit dem Biuretisierungsmittel in Amine, also die Vorstufe der Isocyanate überführt wird. Es wurden deshalb Verfahren vorgeschlagen, Diisocyanate direkt mit ihren Diamin-Vorstufen zu den Biuret-Polyisocyanaten umzusetzen. Dabei werden jedoch, insbesondere im Falle des technisch wichtigsten Vertreters, 1,6-Diioscyanatohexan, auch bei Einsatz hochentwickelter Mischverfahren infolge der hohen Reaktivität der Diamine schwerlösliche Polyharnstoffe erhalten, zu deren Auflösung eine starke Temperaturbelastung notwendig ist, verbunden mit einer Verschlechterung der Farbqualität sowie dem vermehrten Auftreten von Nebenprodukten. Neben dimeren Uretdionen und trimeren Isocyanuraten treten dabei auch Carbodiimide bzw. Folgeprodukte von Carbodiimiden auf, die die Monomerenstabilität des Endproduktes ungünstig beeinflussen.

Herabgesetzt werden kann die Tendenz zur Bildung von schwerlöslichen Polyharnstoffen durch Einsatz von Diaminen, deren Kohlenstoffgerüst nicht den eingesetzten Diisocyanaten entspricht, und die auf geeignete Weise in ihrer Reaktivität deutlich herabgesetzt sind, z.B. durch sterische Hinderung. Die Produkte enthalten u.a. jedoch einen hohen Anteil an monomeren Diisocyanaten, die aus den eingesetzten Diaminen entstehen und die nicht durch Dünnschichtdestillation zu entfernen sind.

Beim Einsatz von ω,ω'-Diaminopolyethern werden wohl flüssige, biurethaltige Polyisocyanate erhalten; diese Lösung ist jedoch wegen der zusätzlichen Synthese des Biuretisierungsmittels aufwendig. Außerdem führen die in diesen Produkten vorhandenen Ethergruppierungen zu einem schlechten Bewitterungsverhalten daraus hergestellter Lackfilme.

Vermieden werden kann das Auftreten von Polyharnstoffen durch Einsatz von Monoaminen oder N,N'-disubstituierten Harnstoffen. Hierbei müssen jedoch die aus diesen Biuretisierungsmitteln resultierenden, leichtflüchtigen Monoisocyanate aus dem Reaktionsgemisch entfernt werden. Dies ist jedoch selbst bei hoher Temperatur wegen der notwendigen Äquilibrierungsreaktionen nur unvollständig möglich.

Bei der Umsetzung von Diisocyanaten mit Ameisensäure lassen sich zwar unter schonenden Bedingungen Produkte mit guter Farbqualität herstellen, die jedoch noch in hohem Ausmaß N-Formylgruppierungen enthalten. Um ein Polyisocyanat mit überwiegender Biuretstruktur zu erhalten, sind Reaktionstemperaturen von mehr als 160°C über längere Zeit nötig, was zu einer deutlichen Gelbfärbung der Produkte führt. Das Biuretisierungsmittel wird darüber hinaus verbraucht unter Freisetzung von giftigem Kohlenmonoxid, wodurch in verstärktem Maße Abluftprobleme hervorgerufen werden.

Ebenfalls vorgeschlagen wurden Verfahren, bei denen Ammoniak- bzw. Amin-Alkohol-Gemische eingesetzt werden.

Abgesehen von anderen Nachteilen resultieren aus solchen Verfahrensweisen Produkte veränderter Struktur mit unterschiedlichem Eigenschaftsbild. Das gleiche gilt für Produkte, bei deren Herstellung Diisocyanate mit Ammoniak umgesetzt werden.

Es wurde nun gefunden, daß man Polyisocyanate mit Biuretstruktur in ausgezeichneter Farbqualität und mit guten Monomerenstabilität durch Umsetzung von aliphatischen Diisocyanaten im Überschuß mit Wasser in Gegenwart von speziellen trisubstituierten Essigsäuren oder von Anhydriden dieser Carbonsäuren herstellen kann.

Gegenstand der Erfindung ist somit ein Verfahren zur Herstellung von Polyisocyanaten mit Biuretstruktur durch Umsetzung von aliphatischen Diisocyanaten mit Wasser bei erhöhter Temperatur gegebenenfalls unter Überdruck, wobei die Umsetzung gegebenenfalls in Gegenwart von mit Wasser mischbaren Lösungsmitteln erfolgt, und wobei gegebenenfalls Lösungsmittel, nicht umgesetztes Ausgangsdiisocyanat und sonstige flüchtige Bestandteile von dem Biuretpolyisocyanat nach Beendigung der Biuretisierungsreaktion durch Destillation und/oder Extraktion entfernt werden, dadurch gekennzeichnet, daß man die Umsetzung in Gegenwart von

a) α, α, α-trisubstituierten Essigsäuren, die neben der Carboxylgruppe keine weitere gegenüber Isocyanatgruppen reaktionsfähige Gruppe aufweisen,

und/oder von

b) Anhydriden von Säuren der unter a) genannten Art

durchführt, wobei pro Mol Wasser 0 bis 0,39 Mol trisubstituierte Essigsäure a) und/oder 0 bis 2 Mol Anhydrid b) einer trisubstituierten Essigsäure eingesetzt werden, mit der Maßgabe, daß die Gesamtmenge von a) und b) pro Mol Wasser mindestens 0,02 und höchstens 2 Mol beträgt.

Für das erfindungsgemäße Verfahren werden lineare oder verzweigte aliphatische Diisocyanate mit 4 bis 30, vorzugsweise 5 bis 12 Kohlenstoffatomen im Kohlenwasserstoffrest, die gegebenenfalls auch eine oder mehrere Estergruppierungen aufweisen können, verwendet.

Genannt sein beispielsweise: 1,4-Diisocyanato-butan, 1,5-Diisocyanato-pentan, 1,6-Diisocyanato-hexan, 1,8-Diisocyanato-octan, 1,10-Diisocyanato-decan, Isomerengemisch aus 2,2,4-Trimethyl-1,6-diisocyanatohexan und 2,4,4-Trimethyl-1,6-diisocyanatohexan, 2-Methyl-1,5-diisocyanato-pentan, 2,2-Dimethyl-diisocyanato-pentan, ω'-Isocyanato-capronsäure-(2-Isocyanatoethyl)-ester, ω, ω'-Diisocyanato-capronsäureethylester.

Besonders bevorzugt wird beim erfindungsgemäßen Verfahren 1,6-Diisocyantohexan eingesetzt. Die Diisocyanate können in handelsüblicher Qualität verwendet werden, so daß eine Vorreinigung oder Temperung nicht erforderlich ist.

Bei den erfindungswesentlichen Zusatzmitteln handelt es sich um

a) α, α, α-trisubstituierte Essigsäuren, die neben der Carboxylgruppe keine gegenüber Isocyanatgruppen reaktionsfähige Gruppe aufweisen, und deren α-Kohlenstoffatom, da trisubstituiert, nicht mit Wasserstoff verknüpft ist; und/oder um

b) Anhydride derartiger Säuren.

Geeignete trisubstituierte Essigsäuren bzw. Essigsäureanhydride sind beispielsweise Verbindungen der allgemeinen Formeln

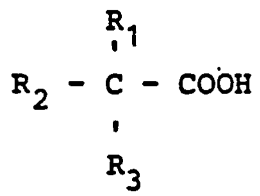

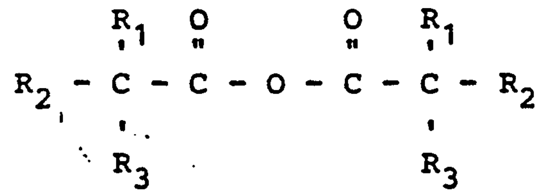

in welchen

$R_1$, $R_2$ und $R_3$ für gleiche oder verschiedene Reste stehen und Alkyl-, Alkoxy- oder Alkoxyalkylgruppen bedeuten, wobei auch zwei der Substituenten zusammen mit dem α-ständigen Kohlenstoffatom der Essigsäure bzw. des Essigsäureanhydrids einen cycloaliphatischen Ring bilden können.

Bevorzugte Verbindungen der genannten allgemeinen Formeln sind solche, für welche die Reste $R_1$, $R_2$ und $R_3$ für Alkylreste stehen, wobei die Summe der Kohlenstoffatome der Reste $R_1$, $R_2$ und $R_3$ 3 bis 6, vorzugsweise 3 oder 4 und insbesondere 3 beträgt. Geeignet sind beispielsweise 2,2,2-Trimethylessigsäure (Pivalinsäure), 2,2-Dimethylbuttersäure, 2,2,3-Trimethylbuttersäure, 2-Methyl-2-methoxymethyl-propionsäure oder 1-Methylcyclopropancarbonsäure. Auch Methacrylsäure ist eine erfindungsgemäß geeignete trisubstituierte Essigsäure, da sie der obengenannten Bedingung genügt, daß das α -Kohlenstoffatom mit keinem Wasserstoffatom verknüpft ist. Wie ausgeführt, können anstelle oder in Kombination mit den beispielhaft genannten trisubstituierten Essigsäuren auch die entsprechenden Säureanhydride eingesetzt werden. Vorzugsweise wird Trimethylessigsäure und/oder Trimethylessigsäureanhydrid verwendet. Grundsätzlich ist es auch denkbar, eine geeignete trisubstituierte Essigsäure zusammen mit einem Anhydrid einer anderen geeigneten trisubstituierten Essigsäure zu verwenden.

Bei der Durchführung des erfindungsgemäßen Verfahrens wird die Menge an Wasser, trisubstituierter Essigsäure und/oder deren Anhydrid so bemessen, daß folgende Voraussetzungen erfüllt sind:

1. Das Molverhältnis von Ausgangsdiisocyanat zu "Gesamtwasser" beträgt 3:1 bis 20:1, vorzugsweise 5:1 bis 12:1. Unter "Gesamtwasser" ist hierbei nicht nur das in Substanz als Biuretisierungsmittel zugesetzte Wasser, sondern auch das in Form der Säure eingeführte potentielle Wasser zu verstehen, das bei vollständiger Überführung der Säure in ihr Anhydrid unter Wasserabspaltung gebildet würde. Die Angabe "1 Mol (potentielles) Wasser" entspricht demzufolge der Angabe "2 Mol trisubstituierte Essigsäure".

2. Die trisubstituierten Essigsäuren und/oder deren Anhydride werden im übrigen in solchen Mengen mitverwendet, daß auf jedes Mol Wasser 0 bis 0,39, vorzugsweise 0 bis 0,25 Mol trisubstituierte Essigsäure und/oder 0 bis 2, vorzugsweise 0 bis 0,5 Mol Anhydrid einer trisubstituierten Essigsäure entfallen, mit der Maßgabe, daß die Gesamtmenge an trisubstituierter Essigsäure und/oder an Anhydrid einer trisubstituierten Essigsäure pro Mol Wasser mindestens 0,02, vorzugsweise mindestens 0,03 Mol und höchstens 2 Mol, vorzugsweise höchstens 0,5 Mol beträgt.

Es kann zweckmäßig sein, das erfindungsgemäße Verfahren in Gegenwart eines mit Wasser zumindest in gewissen Grenzen mischbaren, gegen Isocyanate und Säuren inerten Lösungsmittels durchzuführen.

Als gegebenenfalls mitzuverwendende Lösungsmittel seien beispielhaft benannt: Ether, wie Diisopropylether, Ethylenglykoldimethylether, Diethylenglykoldimethylether, 1,4-Dioxan, Tetrahydrofuran, 1,2-Dimethoxypropan; Ester, wie: Butyrolacton, Ethylenglykolcarbonat, Propylenglykolcarbonat; Ether-Ester, wie Methoxyethylacetat, Ethoxyethylacetat, 1-Methoxypropyl-2-acetat, 2-Methoxypropyl-1-acetat, 1-Ethoxypropyl-2-acetat, 2-Ethoxypropyl-1-acetat; Ketone, wie Aceton, Methylethylketon; Nitrile, wie Acetonitril, Propionitril, Methoxypropionitril; Sulfone, wie Sulfolan, Dimethylsulfon, Diethylsulfon; Phosphorsäureester, wie Trimethylphosphat, Triethylphosphat.

Als weniger bevorzugte Lösungsmittel seien genannt: Tetramethylharnstoff, N-Methylpyrrolidon, Dimethylformamid, Dimethylacetamid.

Das erfindungsgemäße Verfahren wird bei Temperaturen von 50 bis 160°C, vorzugsweise 80 - 140°C, durchgeführt.

Üblicherweise wird das Verfahren bei Normaldruck durchgeführt. Insbesondere bei Mitverwendung von niedrigsiedenden Lösungsmitteln kann das Verfahren natürlich auch bei Drücken von 1 bis 50 bar, vorzugsweise 1 bis 5 bar, durchgeführt werden.

Bei der Herstellung von Biuretpolyisocyanaten unter Verwendung von Wasser als Biuretisierungsmittel

reagiert dieses mit Diisocyanaten über die intermediäre Bildung von Carbamidsäure zu den entsprechenden Aminen und Kohlendioxid. Die gebildeten Amine reagieren mit weiterem Isocyanat in einer äußerst schnellen Reaktion zu schwerlöslichen Harnstoffen, die in Folge mit überschüssigem Isocyanat zu dem Enprodukt weiterreagieren.

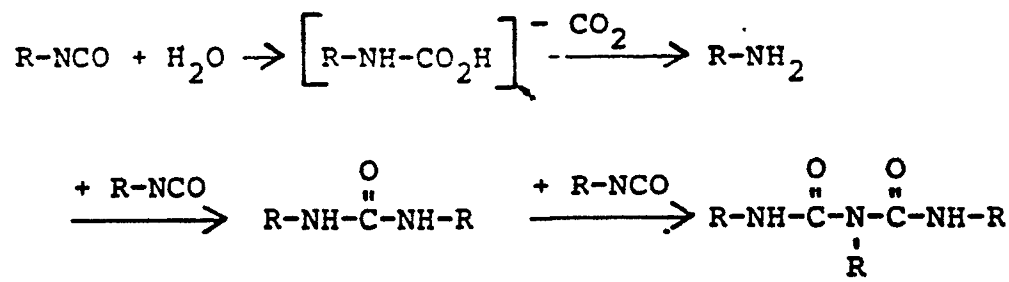

Diese Reaktionsfolge kann, wie bereits geschildert, nur schwer, mit hohem Aufwand und unter Inkaufnahme von Nachteilen so durchgeführt werden, daß klare Produkte mit annehmbarer Farbqualität und einem noch vertretbaren Ausmaß an Nebenprodukten entstehen. Der Hauptgrund für das Auftreten von Verfärbung sowie von Nebenprodukten, die die Monomerenstabilität beeinträchtigen, ist die bei allen bisher bekannten Verfahren notwendige hohe Temperaturbelastung des Produktes.

Setzt man Carbonsäuren mit Isocyanaten um, so erhält man in Analogie zur oben beschriebenen Primärreaktion mit Wasser zunächst ein unbeständiges gemischtes Carbamidsäure-Carbonsäure-Anhydrid, das wiederum in Analogie zu der Wasser-Reaktion unter Abspaltung von Kohlendioxid in ein Carbonsäureamid zerfällt. Diese Reaktionsweise (I) wird üblicherweise für die Reaktion von aliphatischen Isocyanaten mit Carbonsäuren in der Literatur beschrieben.

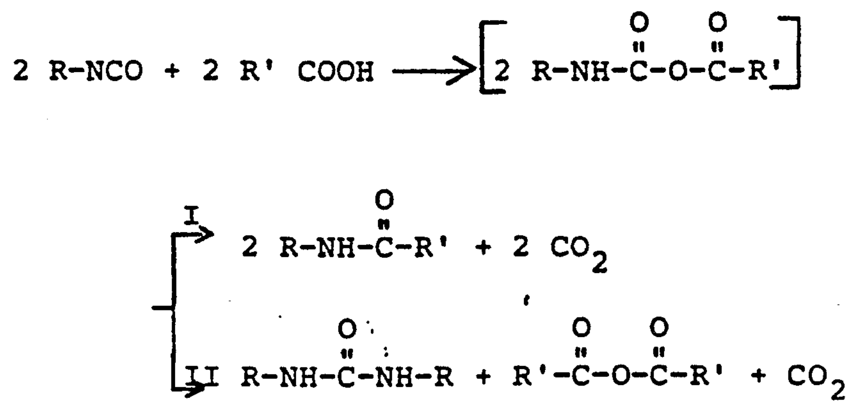

Nur vereinzelt wird, insbesondere für aromatische Isocyanate auch eine alternative Reaktionsweise (II) unter Bildung von Harnstoff, Carbonsäureanhydrid und nur der halben Menge an Kohlendioxid erwähnt.

Zwar wird in der DE-AS 1 174 760 beschrieben, daß im Falle der Reaktion von Ameisensäure mit Isocyanaten ausschließlich die Bildung von Harnstoff unter Abspaltung von Kohlenmonoxid als innerem Anhydrid erfolgt, und wobei die entstehenden Harnstoffe mit weiterem Isocyanat zu Biureten weiterreagieren. Eigene Nacharbeiten sowie die Lehre der DE-OS 2 437 130 zeigen jedoch, daß in den Produkten entsprechend der DE-AS 1 174 760 in sehr hohem Ausmaß N-Formylharnstoff-Gruppierungen anstelle von Biuret-Gruppierungen enthalten sind.

Der erfindungsgemäße Befund liegt nun gerade darin, daß die Reaktion zwischen Diisocyanaten und Wasser zu Harnstoffen und insbesondere auch die Weiterreaktion der gebildeten Harnstoffe zu Biureten in Anwesenheit der erfindungswesentlichen Carbonsäuren und/oder deren Anhydride sehr viel rascher und bei niedrigeren Temperaturen verläuft.

Die erfindungsgemäße Herstellung von Biuretpolyisocyanaten gelingt z.B. schon bei Temperaturen von 80 bis 120°C, ohne daß die intermediär gebildeten Harnstoffe sich anreichern und zu Ausfällungen führen, sondern rasch zu Biureten weiterreagieren. In Abwesenheit der erfindungswesentlichen Säuren und/oder Anhydride führt die Umsetzung von z.B. 1,6-Diisocyanatohexan mit Wasser bei Temperaturen unterhalb 140°C auch in Gegenwart von viel hydrophilem Lösungsmittel zur Ausfällung der intermediär gebildeten Harnstoffe. Die Homogenisierung durch Biuret-Bildung bedarf einer längeren Behandlung bei höheren Temperaturen, z.B. 2 Stunden bei 180°C. Offenbar wirken die erfindungsgemäßen Säuren bzw. Anhydride als Überträger in dem

Sinne, daß aus Säure, die im Falle der Verwendung von Anhydriden aus diesen und Wasser durch Hydrolyse gebildet wird, und dem Diisocyanat ein gemischtes Anhydrid gebildet wird, das mit Wasser unter Bildung von Carbamidsäure und Carbonsäure und insbesondere auch mit bereits gebildetem Harnstoff unter Bildung von Biuret und Carbonsäure reagiert. Aus diesem Grunde ist es auch nicht notwendig, ein Lösungsmittel in großer Menge mitzuverwenden, um ein vollständig homogenes Reaktionsgemisch zu erhalten. Setzt man dagegen 1,6-Diisocyanatohexan ohne, bzw. in Gegenwart von wenig Lösungsmittel und ohne die erfindungsgemäßen Katalysatoren direkt mit Wasser um, so erhält man aufgrund des zweiphasigen Reaktionsgemisches überwiegend schwerlösliche polyharnstoffe, zu deren Auflösung hohe Temperaturen über einen längeren Zeitraum notwendig sind.

Andere als die erfindungswesentlichen Säuren wie beispielsweise Ameisensäure oder Essigsäure können die beschriebene Wirkung nicht oder nur in begrenztem Ausmaß ausüben, da sie zum überwiegenden Teil Säureamide bilden, aus denen nicht die freien Säuren zurückgebildet werden können. Im Falle der Ameisensäure wird zwar die teilweise Bildung von Harnstoffen bzw. Biureten beobachtet. Der in dieser Weise reagierende Anteil der Säure wird dabei jedoch in ihr inneres Anhydrid, Kohlenmonoxid umgewandelt, aus dem unter den Reaktionsbedingungen selbstverständlich keine Ameisensäure durch Hydrolyse erzeugt werden kann.

Das erfindungsgemäße Verfahren kann z.B. wie folgt durchgeführt werden:

Das einzusetzende Diisocyanat wird in einem Rührreaktor, der gegebenenfalls mit einer Meßeinrichtung für das entstehende Kohlendioxid versehen ist, zusammen mit einem Teil oder der Gesamtmenge des gegebenenfalls mitverwendeten Lösungsmittels, bei einer Temperatur von 60 bis 140°, vorzugsweise 80 bis 120° C vorgelegt.

Zu dem Diisocyanat bzw. Diisocyanat-Lösungsmittel-Gemisch werden Wasser, die erfindungsgemäß zu verwendende Säure und/oder deren Anhydrid gemeinsam oder separat, gegebenenfalls zusammen mit einem Lösungsmittel, zudosiert.

Bei der Verwendung von Anhydriden werden diese vorteilhaft zusammen mit dem Diisocyanat und gegebenenfalls einem Lösungsmittel vorgelegt, während bei der Verwendung von Säuren eine synchrone Zudosierung von Säure und Wasser bevorzugt wird.

Wird beispielsweise Pivalinsäure verwendet, führt man die Reaktion vorteilhaft bei 80 bis 100° C durch. Dabei können Pivalinsäure und Wasser zusammen im Gemisch mit einem Lösungsmittel zudosiert werden, wobei die Lösungsmittelmenge so bemessen ist, daß ein homogenes Gemisch entsteht. Die restliche Menge des Lösungsmittels kann dann zusammen mit dem Diisocyanat im Reaktionsgefäß vorgelegt werden.

Soll das bei der Reaktion entstehende oder unverändert im Reaktionsgemisch vorliegende Säureanhydrid anschließend abgetrennt und zur Säure hydrolysiert werden, wird vorteilhafterweise ein Lösungsmittel mitverwendet, dessen Siedepunkt zwischen den Siedepunkten von Diisocyanat und Säureanhydrid liegt. Bei Einsatz von 1,6-Diisocyanatohexan trifft dies beispielsweise für die Lösungsmittel Triethylenglykoldimethylether, Diethylenglykolmonomethyletheracetat und Phosphorsäuretriethylester zu. Dann kann bei der Destillation das Pivalinsäureanhydrid vollständig mit Anteilen des Lösungsmittels aus dem Reaktionsgemisch entfernt werden und ohne weitere Reinigung zur Säure hydrolysiert werden.

Der Siedepunkt des zu verwendenden Lösungsmittels ist dagegen unkritisch bei Verwendung von Carbonsäureanhydriden, da in diesem Fall das Anhydrid zusammen mit dem Diisocyanat vorgelegt werden kann und nach Beendigung der Reaktion zusammen mit überschüssigem monomeren Diisocyanat von Biuret-polyisocyanat abgetrennt und ohne Aufarbeitung wieder verwendet werden kann.

Die verwendeten Lösungsmittelmengen sind in jedem Fall deutlich geringer als z.B. bei dem Verfahren entsprechend der Lehre der DE-OS 2 808 801, bei dem die Menge des eingesetzten Lösungsmittels etwa die Hälfte der Menge des Isocyanats betragen muß, damit ein homogenes Reaktionsgemisch erhalten wird. Für das erfindungsgemäße Verfahren sind dagegen bereits 1/3 bis 1/10 der dort benötigten Mengen völlig ausreichend, wodurch der Destillationsaufwand deutlich verringert wird.

Wird die Reaktion statt durch Säuren durch deren Anhydride katalysiert, sind zum Erreichen vergleichbarer Reaktionszeiten und Temperaturen etwa die doppelte molare Menge an Anhydrid notwendig. Um die für eine ausreichende Reaktionsgeschwindigkeit notwendige Menge an Anhydrid in Grenzen zu halten, ist es daher vorteilhaft, bei einer gegenüber der mit Säure katalysierten Reaktion leicht erhöhten Temperatur von etwa 100 bis 120°C zu arbeiten. Will man gleiche Mengen Anhydrid wie Säure einsetzen, ist es notwendig, eine Reaktionstemperatur von ca. 120°C zu wählen, um die gleiche Reaktionsgeschwindigkeit zu erreichen wie bei der säure-katalysierten Reaktion.

Selbstverständlich können auch die säurekatalysierten Umsetzungen bei höheren Temperaturen durchgeführt werden; eine Temperaturerhöhung bietet hier jedoch keinerlei Vorteile, sondern beeinträchtigt, wenn auch nur gering, durch das Auftreten von Nebenprodukten, insbesondere von dimeren Isocyanaten (Uretdionen), die Qualität der Produkte.

Ebenfalls möglich ist eine Verfahrensweise, bei dem Diisocyanat, Säure und/oder Anhydrid sowie Wasser und gegebenenfalls Lösungsmittel bei niedriger Temperatur gemeinsam vorgelegt werden und die Reaktion durch Aufheizen des Gemisches gestartet wird. Bei Verwendung von Säure als Katalysator beginnt die Reaktion bei etwa 50 bis 60°C, bei der Anhydrid-Katalyse sind etwas höhere Temperaturen (ca. 80°C) nötig. Unvermeidlich treten jedoch intermediär Harnstoffausfällungen auf, zu deren Wiederauflösung unter Biuretbildung höhere Temperaturen und längere Zeiten notwendig sind. Diese Verfahrensweise ist deshalb weniger bevorzugt.

Im Falle der Mitverwendung niedrigsiedender Lösungsmittel, die insbesondere bei Anhydrid-katalysierter Verfahrensweise vorteilhaft eingesetzt werden können, kann es zweckmäßig sein, zur Vermeidung von Lösungsmittel- oder Wasserverlusten unter Überdruck zu arbeiten. Vorteilhaft wird dabei der maximale Druck während der Reaktion durch geeignete Maßnahmen, z.B. durch ein Druckhalteventil, auf 6 bar begrenzt, da bis zu diesem Druck die Benutzung üblicher technischer Apparate problemlos möglich ist. Die Reaktion kann selbstverständlich auch unter höheren Drücken, z.B. unter dem vollständigen Eigendruck des bei der Reaktion entstehenden Kohlendioxids durchgeführt werden, wobei je nach Temperaturführung und Befüllungsgrad des Reaktors Drücke bis zu maximal 20 bar auftreten können. In solchen Fällen sind jedoch spezielle Hochdruckapparaturen notwendig.

Das Verhältnis von NCO-Gruppen zu Wasser und gegebenenfalls Säure kann, wie bereits ausgeführt, in weiten Grenzen variiert werden. Es bestimmt die Oligomerenverteilung des resultierenden Biuret-Polyisocyanats und damit maßgebliche Eigenschaften des Produktes, z.B. Isocyanatgehalt und Viskosität. So weisen zwei handelsübliche Biuret-Polyisocyanate Viskositäten von 10.000 bzw. 2.500 mPa.s bei 23°C auf. Diese Viskositäten werden erfindungsgemäß beispielsweise erreicht, wenn entsprechend dem erfindungsgemäßen Verfahren 3/11 bzw. 1/6 der eingesetzten NCO-Gruppen mit Säure oder Säure/Wasser-Gemischen zu Biureten reagieren.

Selbstverständlich können durch größere Überschüsse an Ausgangsdiisocyanat Endprodukte von noch geringerer Viskosität hergestellt werden. Das Verfahren wird jedoch infolge des höheren Destillationsaufwandes, der geringen Raum-Zeit zunehmend unökonomisch. Darüber hinaus werden durch den größeren Isocyanatüberschuß vermehrt Nebenprodukte gebildet, die nach dem Entfernen dieses Überschusses im Endprodukt angereichert werden.

Andererseits wird die Variationsbreite begrenzt durch das Auftreten von Produkten mit extrem hohen Viskositäten, die zunehmend mit unpolaren Lösungsmitteln, wie sie für die Verarbeitung von Polyisocyanaten eingesetzt werden, unverträglicher werden. Bei Umsetzung von mehr als der Hälfte der NCO-Gruppen des ursprünglich vorliegenden Diisocyanats (Molverhältnis Diisocyanat: "Gesamtwasser" $< 3:1$) muß natürlich mit der Bildung unlöslicher Gele gerechnet werden.

Wird Säureanhydrid als Katalysator verwendet, kann dieses zusammen mit überschüssigem Diisocyanat und gegebenenfalls vorhandenem Lösungsmittel destillativ, vorzugsweise durch Dünnschichtverdampfung, entfernt werden. Das erhaltene Destillat kann dann ohne weitere Reinigungsschritte wiederverwendet werden, wobei geringfügige Verluste durch Zusatz entsprechender Menge an Anhydrid oder vorteilhalter Säure ausgeglichen werden können.

Soll jedoch das Anhydrid nach Hydrolyse in Form der freien Säure wiederverwendet werden, ist es vorteilhaft, Diisocyanat, Carbonsäureanhydrid und gegebenenfalls vorhandenes Lösungsmittel aus dem Reaktionsgemisch destillativ zu entfernen. Wurde kein Lösungsmittel verwendet, ist es vorteilhaft, das gebildete Säureanhydrid, wenn es niedriger siedet als das eingesetzte Diisocyanat, zunächst durch Destillation im Vakuum dem Reaktionsgemisch weitestgehend zu entziehen. In der Praxis kann mit vertretbarem Aufwand der Anteil an Anhydrid, der 1 bis 3 Gew.-% des Diisocyanats überschreitet, in reiner Form abdestilliert werden. Das restliche Anhydrid verbleibt somit im Reaktionsgemisch und wird in einer anschließenden Dünnschichtdestillation zusammen mit dem überschüssigen Diisocyanat vom Biuret-Polyisocyanat abgetrennt. Da das im überschüssigen Diisocyanat verbleibende Anhydrid für das erfindungsgemäße Verfahren in keiner Weise schädlich ist, kann das erhaltene Gemisch ohne weitere Maßnahmen wiederverwendet werden. Das vorher abgetrennte Anhydrid kann in einfacher Weise durch Erhitzen mit Wasser zur Säure hydrolysiert und ebenfalls wiederverwendet werden. Dabei ist eine vollständige Hydrolyse des entstandenen Anhydrids nicht notwendig, da das verbleibende Anhydrid ebenfalls katalytisch wirksam ist.

Die Verfahrensweise, gebildetes Säureanhydrid vollständig aus der Reaktionslösung destillativ zu entfernen, liefert ein mit Diisocyanat verunreinigtes Produkt, das, wenn es zwecks Wiederverwendung zur Säure hydrolysiert werden soll, in einem weiteren Destillationsschritt gereinigt werden muß. Bei den relativ kleinen Mengen ist es dabei vorteilhaft, das bei mehreren Ansätzen anfallende Anhydrid zu sammeln und gemeinsam aufzuarbeiten.

Falls das Anhydrid der eingesetzten Carbonsäure höher siedet als das eingesetzte Diisocyanat, werden beide zusammen durch Dünnschichtdestillation entfernt, wobei die Gesamtmenge des Diisocyanats erneut destilliert werden muß, wenn das Anhydrid anschließend zur Säure hydrolysiert werden soll. Da darüber hinaus Restmengen des Anhydrids immer im fertigen Produkt verbleiben, wird diese Verfahrensweise weniger bevorzugt und es werden vorteilhaft solche Säuren eingesetzt, deren Anhydride niedriger sieden als die verwendeten Diisocyanate.

Wird ein Lösungsmittel mitverwendet, kann dieses, je nach Siedepunkt, separat bzw. zusammen mit dem Anhydrid oder zusammen mit dem überschüssigen Diisocyanat aus dem Reaktionsgemisch entfernt und in den Prozeß zurückgeführt werden. Wenn es zusammen mit dem gebildeten Säureanhydrid isoliert wird, bedarf es bei der sich gegegebenenfalls anschließenden Hydrolyse des Anhydrids keiner Abtrennung des Lösungsmittels. Der Einsatz eines Lösungsmittel/Anhydrid-Gemisches zur Hydrolyse kann darüber hinaus vorteilhaft sein, da das Lösungsmittel als Lösungsvermittler zwischen Wasser und dem mit Wasser unter Umständen nicht mischbaren Anhydrid wirkt.

Selbstverständlich eignet sich das Verfahren in hervorragender Weise auch für eine kontinuierliche Durchführung. In diesem Fall wird beispielsweise in den ersten von 4 bis 6 kaskadenförmig hintereinander geschalteten Rührreaktoren Diisocyanat und Carbonsäure und/oder Anhydrid zusammen mit Wasser und

gegebenenfalls einem Lösungsmittel separat oder als Gemisch so zudosiert, daß sich bis zum Verlassen des letzten Reaktors eine Verweilzeit von 2 bis 8 Stunden ergibt. Dabei kann die Temperatur in den einzelnen Reaktoren entweder gleichförmig 100 bis 140°C betragen oder von 60 bis 140°C, besser 80 bis 120°C ansteigen.

Je nach den Siedepunkten von Diisocyanat, gebildetem Säureanhydrid und gegebenenfalls mitverwendetem Lösungsmittel wird das Reaktionsgemisch, wenn das gebildete Anhydrid zur Säure hydrolysiert und wiederverwendet werden soll, entweder zuerst über eine kontinuierlich zu betreibende Destillationskolonne zur Abtrennung des Anhydrids, gegebenenfalls zusammen mit dem Lösungsmittel, geleitet und anschließend das Biuret-Polyisocyanat durch Dünnschichtdestillation oder Extraktion von überschüssigem Diisocyanat sowie Resten von Anhydrid und gegebenenfalls vorhandenem Lösungsmittel befreit, oder zunächst das Biuret-Polyisocyanat durch gemeinsame Dünnschichtverdampfung von überschüssigem Diisocyanat, Anhydrid und Lösungsmittel befreit und das Brüdenprodukt anschließend über eine Kolonne in Diisocyanat und Anhydrid aufgetrennt, wobei das gegebenenfalls mitverwendete Lösungsmittel entweder separat oder zusammen mit einer der anderen Komponenten abgetrennt werden kann. Das so gewonnene, gegebenenfalls im Gemisch mit Lösungsmittel vorliegende Anhydrid wird anschließend kontinuierlich oder diskontinuierlich vollständig oder partiell zur Säure hydrolysiert und, ebenso wie das überschüssige Diisocyanat, in den Prozeß zurückgeführt.

Beispielsweise würde bei der bevorzugten Reaktion von 1,6-Diisocyanatohexan mit Wasser und Pivalinsäure zunächst das gebildete Pivalinsäureanhydrid über eine Kolonne abgetrennt und der Hydrolyse zugeführt und anschließend das Biuret-Polyisocyanat durch Dünnschichtverdampfung von überschüssigem 1,6-Diisocyanatohexan befreit.

Wird dagegen das Anhydrid selbst als Katalysator verwendet, kann es zusammen mit überschüssigem Diisocyanat und gegebenenfalls mitverwendetem Lösungsmittel durch Dünnschichtdestillation abgetrennt und das erhaltene Gemisch ohne weitere Reinigungsschritte wieder in den Prozeß zurückgeführt werden.

Die nach dem erfindungsgemäßen Verfahren hergestellten Polyisocyanate mit Biuret-Struktur zeichnen sich durch hohe Farbqualität sowie gute Lagerstabilität aus und sind weitgehend frei von Nebenprodukten. Sie eignen sich in hervorragender Weise zur Herstellung von lichtechten und extrem witterungsbeständigen Lacken mit höchster Glanzhaltung.

Das erfindungsgemäße Verfahren wird durch die nachfolgenden Beispiele näher erläutert. Prozentangaben beziehen sich auf Gewichtsprozente.

**Beispiel 1**

In einem 6 l-Vierhalskolben mit Kontaktthermometer, Rührer und Rückflußkühler wurden 5040 g (30 Mol) Hexamethylendiisocyanat bei 90°C vorgelegt. Aus zwei separaten Tropftrichtern wurden im Verlauf von 70 Minuten 73,8 g (4,1 Mol) dest. Wasser und 183 g (1,8 Mol) geschmolzene Pivalinsäure unter gutem Rühren synchron zugetropft. Kurze Zeit nach Beginn des Zutropfens setzte eine stetige $CO_2$-Entwicklung ein; nach beendeter Zugabe wurden mit einer Gasuhr korr. 85,1 1 (76 % d.Th.) gemessen. Nun wurde noch 30 Min. bei 100°C und 60 Min. bei 120°C nachgerührt. Danach war die Gasentwicklung beendet (korr. 109 1 = 97 % d.Th.). Der NCO-Gehalt der hellen Biuret-Rohlösung betrug 37,1 %. Die Lösung wurde durch Filtration von geringfügigen Mengen Feststoff befreit und durch Dünnschichtdestillation aufgearbeitet. Man erhielt 205 g eines Biuretpolyisocyanats mit folgenden Eigenschaften:

NCO-Gehalt: 22,5 %
Viskosität bei 25°C: 7300 mPa.s
APHA-Farbzahl: 20 - 30
Monomerengehalt: 0,15 %
Nach Lagerung (6 Wochen,
50°C): 0,2 %

Das Dünnschichtdestillat (2980 g) enthielt laut gaschromatographischer Analyse 152 g (91 % d.Th.) Pivalinsäureanhydrid. Es wurde ohne weitere Behandlung für den nächsten Versuch eingesetzt.

**Beispiel 2**

2980 g Destillat aus Beispiel 1, das 2828 g (16,8 Mol) Hexamethylendiisocyanat und 152 g (0,82 Mol) Pivalinsäureanhydrid enthielt, wurden zusammen mit weiteren 3226 g (19,2 Mol) Hexamethylendiisocyanat und 500 ml Phosphorsäuretriethylester bei 120°C vorgelegt. Im Verlauf von 60 Min. wurden 72 g (4 Mol) destilliertes Wasser zugetropft und dann noch weitere 80 Min. bei der gleichen Temperatur nachgerührt. Danach waren korr. 87,6 1 (97,8 % d.Th.) $CO_2$ entstanden. Über eine Füllkörperkolonne (50 cm Höhe, 5 cm Durchmesser) mit Rückflußteiler (Rücklaufverhältnis etwa 1:1) wurde nun bei 15 mm eine Fraktion von 200 g abdestilliert, die nach GC-Analyse 141 g (93 % d.Th.) Pivalinsäureanhydrid und 58 g Triethylphosphat enthielt. Hexamethylendiisocyanat war nur in Spuren enthalten. Durch kurzes Aufkochen mit der äquimolaren Menge Wasser wurde eine Pivalinsäurelösung gewonnen, die für weitere Ansätze verwendet werden konnte.

Das erhaltene, völlig klare Roh-Biuret wurde durch Dünnschichtdestillation aufgearbeitet und ergab 1930 g

eines Biuret-Polyisocyanats mit folgenden Eigenschaften:
NCO-Gehalt: 23,7 %
Viskosität bei 25° C: 2350 mPa.s
APHA-Farbzahl: 20
Monomerengehalt: <0,1 %

**Beispiel 3**

1680 g (10 Mol) Hexamethylendiisocyanat wurden zusammen mit 100 ml 1,4-Dioxan bei 100° C vorgelegt und im Verlauf von 40 Min. eine Lösung von 32,4 g (1,8 Mol) destilliertes Wasser und 40,8 g (0,4 Mol) Pivalinsäure in 100 ml Dioxan zugetropft. Nach weiteren 60 Min. bei 120° C war unter Freisetzung der berechneten Menge $CO_2$ eine klare Biuret-Rohlösung mit einem NCO-Gehalt von 35,2 % (berechnet 35,5 %) entstanden. Nach Abdestillieren des Lösungsmittels im Vakuum wurde das Produkt in einer Extraktionskolonne mit n-Hexan extrahiert. Man erhielt ein Biuretpolyisocyanat mit folgenden Eigenschaften:
NCO-Gehalt: 21,5 %
Viskosität bei 25° C: 15800 mPa.s
APHA-Farbzahl: 10 - 20
Monomergehalt: 0,13 %
Nach Lagerung (6 Wochen, 50° C): 0,17 %.

**Vergleichsbeispiel I**

Es wurde wie in Beispiel 3 verfahren; anstelle der Pivalinsäure wurde die gleiche Molmenge Ameisensäure eingesetzt. Nach Freisetzen von etwa der Hälfte der berechneten Menge Kohlendioxid begann sich die Mischung einzutrüben. Im weiteren Verlauf der Reaktion entstand ein voluminöser, flockiger Niederschlag von Harnstoff-Zwischenprodukten. Nach beendeter Gasentwicklung wurde das Gemisch 3 Stunden lang auf 142° C (Rückfluß des Dioxans) erhitzt, jedoch ging der Niederschlag dabei nicht in Lösung. Auf eine weitere Aufarbeitung wurde daher verzichtet.

**Vergleichsbeispiel II**

Beispiel 3 wurde wiederholt; anstelle der Pivalinsäure wurde die gleiche Molmenge Essigsäure eingesetzt. Anders als im Vergleichsbeispiel I trat hier nur eine sehr schwache Trübung auf. Jedoch war bald nach Beginn der Reaktion eine deutliche Gelbfärbung festzustellen, die sich bis zum Ende der Reaktion verstärkte. Da die Biuret-Rohlösung eine APHA-Farbzahl von über 120 aufwies, wurde auf eine Aufarbeitung verzichtet.

**Vergleichsbeispiel III**

1008 g (6 Mol) Hexamethylendiisocyanat, 12,75 g (0,125 Mol) Acetanhydrid und 100 ml 1,4-Dioxan wurden bei 120° C vorgelegt und im Verlauf von 40 Min. mit 18 g (1 Mol) destilliertem Wasser versetzt. Nach einer weiteren Stunde bei 120° C war die Reaktion beendet. Kurze Zeit nach Beginn der Wasserzugabe war eine deutliche Gelbfärbung festzustellen, die sich bis zum Ende der Reaktion laufend verstärkte. Da die APHA-Farbzahl der Rohlösung 180 betrug, wurde auf eine Aufarbeitung verzichtet.

Erhöhte man in einem weiteren Ansatz die Menge Acetanhydrid auf 51 g (0,5 Mol) und senkte die Temperatur der Wasserzugabe auf 90° C, so wurde eine gleich stark gefärbte Biuret-Rohlösung erhalten, die durch Harnstoff-Ausfällungen getrübt war. Auf eine Aufarbeitung wurde deshalb auch hier verzichtet.

**Beispiel 4**

1008 g (6 Mol) 3-Methylpentan-1,5-diisocyanat wurden bei 90° C vorgelegt und eine Lösung von 16,2 g (0,9 Mol) destilliertem Wasser und 17,2 g (0,2 Mol) Methacrylsäure in 50 g Aceton innerhalb 40 Min. zugetropft. Nach weiteren 60 Min. bei 110° C war die Gasentwicklung beendet (korr. 22,3 l = 99,5 % d.Th.).

Nach Abziehen des Lösungsmittels im Vakuum und Entfernen des Diisocyanatüberschusses durch Dünnschichtdestillation wurden 405 g eines Biuretpolyisocyanats mit folgenden Eigenschaften erhalten:

NCO-Gehalt: 24,4 %
Viskosität bei 25° C: 29800 mPa.s
APHA-Farbzahl: 30 - 40
Monomerengehalt: 0,25 %

**Beispiel 5**

1260 g (6 Mol) Trimethyl-hexan-1,6-diisocyanat (Isomerengemisch 2,2,4- und 2,4,4-) wurden bei 60° C mit 200 ml 1,2-Dimethoxyethan, 8,1 g (0,45 Mol) Wasser und 11,6 g (0,1 Mol) 2,2-Dimethylbuttersäure versetzt und weiter bei der gleichen Temperatur gerührt. Nach einigen Minuten begann eine schwache Kohlendioxidentwicklung. Im Verlauf von 5 Stunden entstanden korr: 19,6 l $CO_2$ (87,5 % d.Th.). Zu diesem Zeitpunkt war der Ansatz durch flockige Ausfällungen getrübt. Nun wurde im Verlauf von 3 Stunden die Temperatur schrittweise auf 140° C erhöht. Dabei ging der Niederschlag allmählich in Lösung und die $CO_2$-Menge erhöhte sich auf korr: 22,6 1 (101 % d.Th.). Dann wurde bei 0,2 mm eine Fraktion von 53 g abdestilliert, die laut gaschromatographischer Analyse 9,9 g (92,5 % d.Th.) 2,2-Dimethylbuttersäureanhydrid enthielt. Die Biuret-Rohlösung wurde der Dünnschichtdestillation (Manteltemperatur 170° C, 0,3 mm) unterworfen und lieferte ein Biuretpolyisocyanat mit folgenden Eigenschaften:
NCO-Gehalt: 18,5 %
Viskosität bei 25° C: 11300 mPa.s
APHA-Farbzahl: 40
Monomerengehalt: 0,35 %.

**Beispiel 6**

Ein Gemisch aus 3360 g (20 Mol) Hexamethylendiisocyanat 93 g (0,5 Mol) Pivalinsäureanhydrid und 300 g Phosphorsäuretriethylester wurde bei 120° C vorgelegt und im Verlauf von 40 Min. 36 g (2 Mol) destilliertes Wasser zugetropft. Nach weiteren 2 Stunden bei 120° C wurde durch Dünnschichtdestillation aufgearbeitet. Man erhielt 890 g Biuretpolyisocyanat a.

Das Destillat (2803 g) wurde mit 941 g frischem Hexamethylendiisocyanat auf 3744 g aufgestockt und wie oben mit 35,1 g (1,95 Mol) Wasser reagiert, wobei synchron mit Wasser aus einem separaten Tropftrichter 10,2 g (0,1 Mol) Pivalinsäure zugetropft wurden. (Biuretpolyisocyanat b). Die Verfahrensweise b wurde noch dreimal wiederholt (c - e). Es wurden folgende Ergebnisse erhalten:
Biuretpolyisocyanat: a b c d e
Ausbeute (g): 890 887 885 885 880
NCO-Gehalt (%): 23,9 23,8 23,8 23,7 23,7
Viskosität bei 25° C
(mPa.s): 1850 1860 1860 1880 1910
APHA-Farbzahl: 30-40 30 20-30 20 20
Monomerengehalt (%): 0,15 0,21 0,18 0,05 0,08
Menge Destillat: 2803 2800 2795 2802 2805

Das Destillat des letzten Ansatzes enthielt nach gaschromatographischer Analyse 290 g Phosphorsäuretriethylester und 98 g Pivalinsäureanhydrid, d.h., die Verluste an Katalysator waren etwas geringer als die jeweils zur Kompensation zugefügten Mengen an Pivalinsäure.

**Beispiel 7**

In einem 50 1-Rührautoklaven mit Röhrenbündelkühler wurden 33,6 kg (200 Mol) Hexamethylendiisocyanat, 3,1 Aceton und 744 g (4 Mol) Pivalinsäureanhydrid bei 120° C vorgelegt und über eine Kolbendosierpumpe im Verlauf von 2 Stunden mit 612 g (34 Mol) destilliertem Wasser versetzt. Am oberen Ende des Kühlers war ein Druckhalteventil angebracht, das auf 5 bar eingestellt war.

Nach Zugabe von etwa 15 Mol Wasser (40 Min.) hatte der Kesselinnendruck die vorgegebenen 5 bar erreicht und Kohlendioxid begann zu entweichen. Die Menge wurde über eine Gasuhr verfolgt. Nach beendeter Wasserzugabe wurde noch 2 Stunden bei 120° C gerührt. Die gemessene $CO_2$-Menge (korr. 470,4 1 = 21 Mol) erhöhte sich nach Abkühlen des Kessels auf 80° C und Entspannen auf Umgebungsdruck auf korr. 765 l (34,2 Mol). Die wasserhelle, klare Biuret-Rohlösung wurde über einen Fallfilmverdampfer von Lösungsmittel, pivalinsäureanhydrid und Teilen des Isocyanatüberschusses befreit und anschließend am Dünnschichtverdampfer destilliert. Man erhielt 14,1 kg eines Biuretpolyisocyanats mit folgenden Eigenschaften:
NCO-Gehalt: 22,3 %

Viskosität bei 25° C: 9800 mPa.s
APHA-Farbzahl 20 - 30
Monomerengemisch: 0,35 %
Nach Lagerung: 0,43 %
(12 Wochen, 50° C)

## Patentansprüche

1. Verfahren zur Herstellung von Polyisocyanaten mit Biuretstruktur durch Umsetzung von aliphatischen Diisocyanaten mit Wasser bei erhöhter Temperatur gegebenenfalls unter Überdruck, wobei die Umsetzung gegebenenfalls in Gegenwart von mit Wasser mischbaren Lösungsmitteln erfolgt, und wobei gegebenenfalls Lösungsmittel, nicht umgesetztes Ausgangsdiisocyanat und sonstige flüchtige Bestandteile von dem Biuretpolyisocyanat nach Beendigung der Biuretisierungsreaktion durch Destillation und/oder Extraktion entfernt werden, dadurch gekennzeichnet, daß man die Umsetzung in Gegenwart von

a) α, α, α-trisubstituierten Essigsäuren, die neben der Carboxylgruppe keine weitere gegenüber Isocyanatgruppen reaktionsfähige Gruppe aufweisen,

und/oder von

b) Anhydriden von Säuen der unter a) genannten Art

durchführt, wobei pro Mol Wasser 0 bis 0,39 Mol trisubstituierte Essigsäure a) und/oder 0 bis 2 Mol Anhydrid b) einer trisubstituierten Essigsäure eingesetzt werden, mit der Maßgabe, daß die Gesamtmenge von a) und b) pro Mol Wasser mindestens 0,02 und höchstens 2 Mol beträgt.

2. Verfahren gemäß Anspruch 1, dadurch gekennzeichnet, daß man als Ausgangsdiisocyanat 1,6-Diisocyanatohexan verwendet.

3. Verfahren gemäß Anspruch 1 und 2, dadurch gekennzeichnet, daß man als Komponenten a) und/oder b) Trimethylessigsäure und/oder Trimethylessigsäureanhydrid verwendet.

## Claims

1. A process for the production of polyisocyanates which have a biuret structure by reacting aliphatic diisocyanates with water at elevated temperature, optionally under excess pressure, and the reaction may be carried out in the presence of water-miscible solvents and optionally solvent, unreacted starting diisocyanate and other volatile constituents may be removed by distillation and/or extraction from the biuret polyisocanate on completion of the biuretisation reaction, characterised in that the reaction is carried out in the presence of

a) α, α, α-tri-substituted acetic acids which do not have any other isocyanate-group-reactive groups other than the carboxyl group,

and/or

b) anhydrides of acids of the type mentioned in a) and from 0 to 0.39 mols of tri-substituted acetic acid a) and/or from 0 to 2 mols of anydride b) of a trisubstituted acetic acid may be used per mol of water, on condition that the total quantity of a) and b) per mol of water is at least 0.02 and at most 2 mol.

2. A process according to claim 1, characterised in that 1,6-diisocyanatohexane is used as starting diisocyanate.

3. A process according to claim 1 and 2, characterised in that trimethyl acetic acid and/or trimethyl acetic acid anhydride are used as components a) and/or b).

## Revendications

1. Procédé de préparation de polyisocyanates à structure biuret par réaction de diisocyanates aliphatiques avec de l'eau à température élevée et éventuellement sous une surpression, la réaction ayant eventuellement lieu en présence de solvants miscibles à l'eau et le solvant, le diisocyanate de départ n'ayant pas réagi et les autres constituants volatils étant éventuellement éliminés du biuret-polyisocyanate au terme de la réaction de biurétisation par distillation et/ou par extraction, caractérisé en ce qu'on effectue la réaction en présence

a) d'acides acétiques α, α, α-trisubstitués qui, outre le groupe carboxy, ne comportent aucun autre groupe réactif vis-à-vis des groupes isocyanate et/ou

b) d'anhydrides d'acides du type indiqué sub a), en utilisant, par mole d'eau, 0 à 0,39 mole d'acide acétique trisubstitué a) et/ou 0 à 2 moles d'anhydride b) d'un acide acétique trisubstitué, avec cette réserve que la quantité totale de a) et b) par mole d'eau est d'au moins 0,02 mole et, au maximum, de 2 moles.

2. Procédé selon la revendication 1, caractérisé en ce que, comme diisocyanate de départ, on utilise le 1,6-diisocyanatohexane.

3. Procédé selon les revendications 1 et 2, caractérisé en ce que, comme composants a) et/ou b), on utilise l'acide triméthylacétique et/ou l'anhydride d'acide triméthylacétique.